(19) Europäisches Patentamt
European Patent Office
Office européen des brevets

(11) Veröffentlichungsnummer : **0 411 364 B1**

(12) # EUROPÄISCHE PATENTSCHRIFT

(45) Veröffentlichungstag der Patentschrift : **06.10.93 Patentblatt 93/40**

(51) Int. Cl.$^5$ : **C07D 213/61**

(21) Anmeldenummer : **90113405.6**

(22) Anmeldetag : **13.07.90**

(54) **Verfahren zur Herstellung von chlorierten Nicotinaldehyden.**

(30) Priorität : **26.07.89 DE 3924682**

(43) Veröffentlichungstag der Anmeldung : **06.02.91 Patentblatt 91/06**

(45) Bekanntmachung des Hinweises auf die Patenterteilung : **06.10.93 Patentblatt 93/40**

(84) Benannte Vertragsstaaten : **BE CH DE FR GB IT LI NL**

(56) Entgegenhaltungen : **DE-A- 2 525 442**

(56) Entgegenhaltungen :
**GB-A- 2 002 368**
**HOUBEN-WEYL, METHODEN DER ORGANISCHEN CHEMIE, Band V/3, 1962, Georg Thieme Verlag, Stuttgart, DE; E. FORCHE et al.: "Halogen-Verbindungen"**
**HOUBEN-WEYL, METHODEN DER ORGANISCHEN CHEMIE, Band E3, 1983, Georg Thieme Verlag, Stuttgart, DE; H. BACH et al.: "Aldehyde"**

(73) Patentinhaber : **BAYER AG**
**D-51368 Leverkusen (DE)**

(72) Erfinder : **Jelich, Klaus, Dr.**
**Paul-Ehrlich-Strasse 2**
**D-5600 Wuppertal 1 (DE)**

**EP 0 411 364 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von chlorierten Nicotinaldehyden, welche als Zwischenprodukte für Pharmazeutika oder für Pflanzenschutzmittel bekannt sind.

Es ist bekannt, daß man 6-Chlor-nicotinaldehyd (2-Chlor-5-formyl-pyridin) erhält, wenn man 6-Chlornicotinsäurenitril (2-Chlor-5-cyano-pyridin) mit einer Raney-Nickel-Aluminium-Legierung in wässriger Ameisensäure umsetzt (vergleiche EP-A 1473, Beispiel 21). Das als Ausgangsstoff hierfür einzusetzende 6-Chlornicotinsäurenitril ist jedoch nach bekannter Methodik nicht rein herstellbar (vergleiche US-P 3 391 597).

Es wurde nun gefunden, daß man chlorierte Nicotinaldehyde der allgemeinen Formel (I)

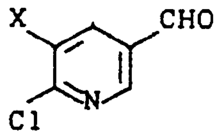

(I)

in welcher

X für Wasserstoff oder Chlor steht,

in sehr guten Ausbeuten und in hoher Reinheit erhält, wenn man zunächst in einer ersten Stufe 6-Alkoxy-nicotinaldehyde der allgemeinen Formel (II)

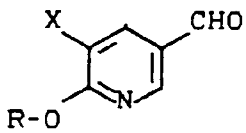

(II)

in welcher

X die oben angegebene Bedeutung hat und

R für Alkyl steht,

mit Chlorierungsmitteln gegebenenfalls in Gegenwart von Verdünnungsmitteln und gegebenenfalls in Gegenwart von Reaktionshilfsmitteln bei Temperaturen von 0° C bis 200° C umsetzt und dann in einer zweiten Stufe die hierbei erhaltenen Dichlormethylpyridine der allgemeinen Formel (III)

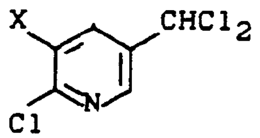

(III)

in welcher

X die oben angegebene Bedeutung hat,

mit Wasser gegebenenfalls in Gegenwart von Säureakzeptoren bei Temperaturen von 0° C bis 110° C umsetzt.

Es ist als überraschend anzusehen, daß bei der Umsetzung von 6-Alkoxy-nicotinaldehyden der Formel (II) mit Chlorierungsmitteln nicht nur die Formylgruppe in eine Dichlormethylgruppe umgewandelt wird, sondern auch zusätzlich ein Austausch der Alkoxygruppe gegen Chlor nahezu quantitativ erfolgt. Auch die glatte Hydrolyse der Dichlormethylverbindungen der Formel (III) ohne Verdrängung eines Chlorsubstituenten am Pyridinring ist als überraschend zu betrachten.

Vorteile des erfindungsgemäßen Verfahrens liegen in der guten Zugänglichkeit der als Ausgangsverbindungen benötigten 6-Alkoxy-nicotinaldehyde (II) sowie in der insgesamt geringen Zahl der Synthesestufen und der Verwendung billiger Synthesechemikalien.

Verwendet man beispielsweise 6-Methoxy-nicotinaldehyd und Phosgen als Ausgangsstoffe, so kann der Reaktionsablauf beim erfindungsgemäßen Verfahren durch das folgende Formelschema skizziert werden:

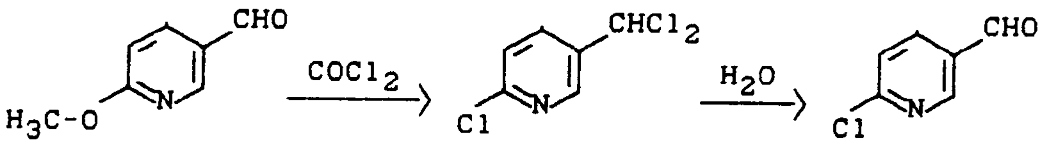


Die als Ausgangsstoffe zu verwendenden 6-Alkoxy-nicotinaldehyde sind durch die Formel (II) allgemein definiert. In Formel (II) steht R für geradkettiges oder verzweigtes Alkyl, vorzugsweise mit 1 bis 6, insbesondere mit 1 bis 4 Kohlenstoffatomen und X steht in jedem Fall für Wasserstoff oder Chlor.

Als Beispiele für die Ausgangsstoffe der Formel (II) seien genannt:
6-Methoxy-, 6-Ethoxy-, 6-Propoxy-, 6-Isopropoxy, 6-Butoxy-, 6-Isobutoxy-, 6-sec-Butoxy- und 6-tert-Butoxynicotinaldehyd, sowie 6-Methoxy-, 6-Ethoxy-, 6-Propoxy-, 6-Isopropoxy-, 6-Butoxy-, 6-Isobutoxy-, 6-sec-Butoxy- und 6-tert-Butoxy-5-chlor-nicotinaldehyd.

6-Alkoxy-nicotinaldehyde (X = H) der Formel (II) sind bekannt und/oder können nach an sich bekannten Verfahren hergestellt werden (vergleiche Eur. J. Med. Chem. - Chim. Ther. 12 (1977), 531 - 536; EP-A 3677; J. Med. Chem. 30 (1987), 1309 - 1313).

5-Chlor-6-alkoxy-nicotinaldehyde (X = Cl) der Formel (II) erhält man aus den 6-Alkoxy-nicotinaldehyden (X = H) der Formel (II) durch Umsetzung mit elementarem Chlor in Gegenwart eines Verdünnungsmittels, wie z. B. Wasser, bei Temperaturen von 0° C bis 100° C (vergleiche Herstellungsbeispiele).

Bei der Durchführung des erfindungsgemäßen Verfahrens kommen als Chlorierungsmittel vorzugsweise anorganische oder organische Säurechloride, wie z. B. Phosphor(III)-chlorid, Phosphor(V)-chlorid, Phosphorylchlorid (Phosphoroxychlorid), Thionylchlorid und Phosgen, in Betracht. Phosgen und Phosphorylchlorid werden als Chlorierungsmittel bevorzugt.

Das erfindungsgemäße Verfahren kann in der ersten Stufe entweder ohne Zusatz eines Verdünnungsmittels in Substanz oder in Gegenwart eines geeigneten Verdünnungsmittels durchgeführt werden. Hierzu gehören insbesondere aliphatische, alicyclische oder aromatische, gegebenenfalls halogenierte Kohlenwasserstoffe, wie beispielsweise Benzol, Xylole, Chlorbenzol, Dichlorbenzole, Petrolether, Hexan, Cyclohexan, Methylcyclohexan, Dichlormethan, Chloroform, Tetrachlormethan oder deren Mischungen und insbesondere Toluol und Dimethylformamid.

Das erfindungsgemäße Verfahren kann in der ersten Stufe gegebenenfalls in Gegenwart eines geeigneten Reaktionshilfsmittels durchgeführt werden. Als solche kommen tertiäre Amine, wie Triethylamin, N,N-Dimethyl-anilin, Pyridin oder 4-Dimethylamino-pyridin in Frage, außerdem auch katalytische Mengen an Formamiden, wie N,N-Dimethyl-formamid oder N,N-Dibutylformamid, oder Metallhalogenide wie Magnesiumchlorid oder Lithiumchlorid.

Die Reaktionstemperaturen können bei der Durchführung des erfindungsgemäßen Verfahrens in der ersten Stufe in einem größeren Bereich variiert werden. Im allgemeinen arbeitet man bei Temperaturen von 0° C bis 200° C, vorzugsweise von 10° C bis 180° C, insbesondere von 50° C bis 160° C.

Zur Durchführung der ersten Stufe des erfindungsgemäßen Verfahrens setzt man soviel Chlorierungsmittel ein, daß pro Molekül der Verbindung der Formel (II) 3 Chloratome eingeführt werden. Im allgemeinen werden auf 1 Mol 6-Alkoxy-nicotinaldehyd der Formel (II) 3 bis 10 Mol, vorzugsweise 3 bis 5 Mol Chlorierungsmittel eingesetzt. Dabei wird das Pyridin-Derivat der Formel (II) vorzugsweise vorgelegt und das Chlorierungsmittel in die Vorlage dosiert.

Es ist aber ebenso möglich, das Chlorierungsmittel vorzulegen und die Ausgangsverbindung der Formel (II) zuzugeben, oder aber die Reaktionspartner simultan einzuspeisen, z.B. in einer kontinuierlich arbeitenden Anlage.

Im Falle von gasförmigen Chlorierungsmitteln, z.B. Phosgen, wird das Chlorierungsmittel vorzugsweise bis zum vollständigen Umsatz in oder über die Vorlage geleitet.

In einer bevorzugten Ausführungsform der ersten Stufe des erfindungsgemäßen Verfahrens wird die Ausgangsverbindung der Formel (II), in Mischung mit einem Verdünnungsmittel und/oder Reaktionshilfsmittel, vorgelegt und Phosgen bis zum vollständigen Umsatz in oder über die Mischung geleitet oder Phosphorylchlorid wird langsam zudosiert. Das Reaktionsgemisch wird bis zum Ende der Umsetzung gerührt und nach üblichen Methoden aufgearbeitet (vergleiche Herstellungsbeispiele).

Als Säureakzeptoren können bei der zweiten Stufe des erfindungsgemäßen Verfahrens ein oder mehrere aller üblicherweise für derartige Umsetzungen verwendbaren Säurebindemittel eingesetzt werden.

Vorzugsweise in Frage kommen Alkalimetallhydroxide wie z. B. Natrium- und Kaliumhydroxid, Erdalkalihydroxide wie z. B. Calciumhydroxid, Alkalihydrogencarbonate und -alkoholate wie Natrium- und Kaliumcarbonat, Natrium- und Kalium-hydrogencarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Natrium- und Kalium-hydrogencarbonat werden als Säureakzeptoren für das erfindungsgemäße Verfahren besonders bevorzugt.

Die Reaktionstemperaturen können bei der zweiten Stufe des erfindungsgemäßen Verfahrens in einem größeren Bereich variiert werden. Im allgemeinen arbeitet man bei Temperaturen von 0° C bis 110° C, vorzugsweise bei Temperaturen von 50° C bis ca. 100° C, insbesondere Rückflußtemperatur.

Das erfindungsgemäße Verfahren wird in beiden Stufen im allgemeinen unter Normaldruck durchgeführt. Es ist jedoch auch möglich, unter erhöhtem oder vermindertem Druck (im allgemeinen von 0,1 bar bis 10 bar) zu arbeiten.

Das bei der zweiten Stufe des erfindungsgemäßen Verfahrens als Reaktionskomponente benötigte Wasser wird im allgemeinen als Verdünnungsmittel und somit in hohem Überschuß eingesetzt.

In einer bevorzugten Ausführungsform der zweiten Stufe des erfindungsgemäßen Verfahrens wird das in der ersten Stufe erhaltene Dichlormethylpyridin der Formel (III) in Wasser suspendiert und nach Einstellen der erforderlichen Reaktionstemperatur ein Säureakzeptor so eindosiert, daß der pH-Wert zwischen 2 und 9, vorzugsweise zwischen 4 und 7, gehalten wird. Nach Ende der Umsetzung wird nach üblichen Methoden aufgearbeitet (vergleiche die Herstellungsbeispiele).

Die nach dem erfindungsgemäßen Verfahren herzustellenden chlorierten Nicotinaldehyde der Formel (I) können als Zwischenprodukte zur Herstellung von Pharmazeutika oder von Pflanzenschutzmitteln verwendet werden (vergleiche DE-OS 24 27 096, EP-A 1473, DE-OS 33 14 196, EP-A 192 060).

Herstellungsbeispiele:

Beispiel 1

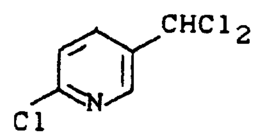

(Stufe 1)

In eine Mischung aus 7,0 g (0,051 Mol) 6-Methoxy-nicotinaldehyd, 21 ml Toluol und 1,6 ml (0,01 Mol) N,N-Dibutylformamid wird bei 75°C bis zum praktisch vollständigen Umsatz (ca. 2 Stunden) Phosgen eingeleitet. Das überschüssige Phosgen wird dann mit Stickstoff ausgeblasen und die Reaktionslösung mit gesättigter Natriumhydrogencarbonat-Lösung gewaschen. Die organische Phase wird mit Natriumsulfat getrocknet und filtriert. Vom Filtrat wird das Lösungsmittel im Wasserstrahlvakuum sorgfältig abdestilliert.

Man erhält 10,0 g eines gelblichen öligen Rückstandes, der durch Säulenchromatographie (Kieselgel/Methylenchlorid) gereinigt wird.

Ausbeute: 8,0 g (80 % der Theorie) 2-Chlor-5-dichlormethyl-pyridin.

Schmelzpunkt: 60° C.

Beispiel 2

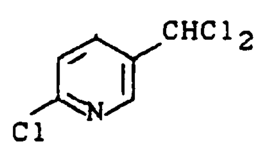

(Stufe 1)

Zu 13,7 g (0,1 Mol) 6-Methoxy-nicotinaldehyd in 130 ml Dimethylformamid werden bei 0° C 30,8 g (0,33 Mol) Phosphorylchlorid tropfenweise gegeben. Das Reaktionsgemisch wird dann 1 Stunde bei 0° C bis 20° C und 4 Stunden unter Rückfluß gerührt, anschließend (nach leichtem Abkühlen) in Eiswasser gegossen und dreimal mit Essigsäureethylester extrahiert. Die vereinigten Extrakte werden mit Wasser gewaschen mit Natriumsulfat getrocknet und filtriert. Vom Filtrat wird das Lösungsmittel im Wasserstrahlvakuum sorgfältig abdestilliert. Man erhält 13,2 g (68 % der Theorie) 2-Chlor-5-dichlormethyl-pyridin als öligen Rückstand, der allmählich kristallisiert. Schmelzpunkt: 60° C.

Beispiel 3

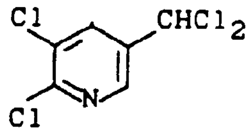

(Stufe 1)

Über eine Mischung aus 10,0 g (0,058 Mol) 5-Chlor-6-methoxy-nicotinaldehyd, 30 ml Toluol und 2 ml (0,012 Mol) N,N-Dibutylformamid wird bei 75° C bis zum praktisch vollständigen Umsatz (ca. 3 Stunden) Phosgen geleitet. Das überschüssige Phosgen wird dann mit Stickstoff ausgeblasen, die Reaktionslösung nach Abkühlen mit Wasser auf etwa das doppelte Volumen verdünnt und mit Natriumcarbonat schwach alkalisch gestellt. Die organische Phase wird abgetrennt, mit Natriumsulfat getrocknet und filtriert. Vom Filtrat wird das Lösungsmittel im Wasserstrahlvakuum sorgfältig abdestilliert.

Man erhält 13 g eines gelblichen öligen Rückstandes, der durch Säulenchromatographie (Kieselgel) gereinigt wird. Ausbeute: 11,0 g (82 % der Theorie)
$n_D^{20}$ : 1,5856.

Beispiel 4

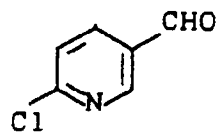

(Stufe 2)

Eine Mischung aus 7,0 g (0,0356 Mol) 2-Chlor-5-dichlormethyl-pyridin und 100 ml Wasser wird zum Rückfluß erhitzt. Durch Zutropfen einer gesättigten Natriumhydrogencarbonat-Lösung wird der pH-Wert zwischen 4 und 7 gehalten. Nach 2 Stunden wird abgekühlt, mit Essigsäureethylester extrahiert und von der organischen Phase das Lösungsmittel im Wasserstrahlvakuum sorgfältig abdestilliert.
Man erhält 4,6 g (95 % der Theorie) 6-Chlor-nicotinaldehyd vom Schmelzpunkt 70° C.

Die Isolierung des Produktes kann statt durch Extraktion auch durch Wasserdampfdestillation erfolgen.

Beispiel 5

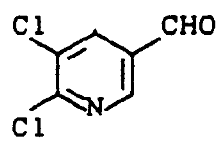

(Stufe 2)

Eine Mischung aus 2,5 g (0,0108 Mol) 2,3-Dichlor-5-dichlormethyl-pyridin und 20 ml Wasser wird zum Rückfluß erhitzt. Durch Zutropfen einer gesättigten Natriumhydrogencarbonat-Lösung wird der pH-Wert zwischen 4 und 7 gehalten.
Nach 6 Stunden wird abgekühlt, mit Essigsäureethylester extrahiert und von der organischen Phase das Lösungsmittel im Wasserstrahlvakuum sorgfältig abdestilliert.

Man erhält 1,4 g (73 % der Theorie) 5,6-Dichlor-nicotinaldehyd vom Schmelzpunkt 65° C.

Ausgangsstoffe der Formel (II)

Beispiel (II-1)

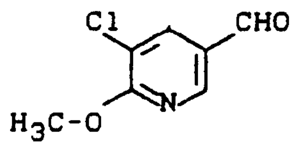

9,5 g (0,069 Mol) 6-Methoxy-nicotinaldehyd werden in 100 ml Wasser suspendiert und bei 45° C wird bis zum praktisch vollständigen Umsatz (ca. 2 Stunden) Chlor eingeleitet. Dann wird nach Abkühlen das kristallin angefallene Produkt durch Absaugen isoliert.
Man erhält 9,4 g (79 % der Theorie) 5-Chlor-6-methoxynicotinaldehyd vom Schmelzpunkt 152° C.

**Patentansprüche**

1. Verfahren zur Herstellung von chlorierten Nicotinaldehyden der allgemeinen Formel (I)

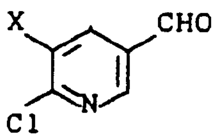

(I)

in welcher
X für Wasserstoff oder Chlor steht,
dadurch gekennzeichnet, daß man in einer ersten Stufe 6-Alkoxy-nicotinaldehyde der allgemeinen Formel (II)

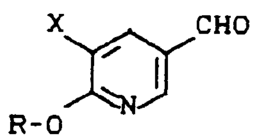

(II)

in welcher
X die oben angegebene Bedeutung hat und
R für Alkyl steht, mit Chlorierungsmitteln gegebenenfalls in Gegenwart von Verdünnungsmitteln und gegebenenfalls in Gegenwart von Reaktionshilfsmitteln bei Temperaturen von 0° C bis 200° C umsetzt und dann in einer zweiten Stufe die hierbei erhaltenen Dichlormethylpyridine der allgemeinen Formel (III)

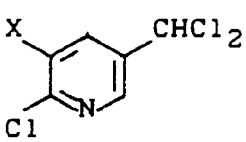

(III)

in welcher
X die oben angegebene Bedeutung hat,
mit Wasser gegebenenfalls in Gegenwart von Säureakzeptoren bei Temperaturen von 0° C bis 110° C umsetzt.

2. Verfahren gemäß Anspruch 1, dadurch gekennzeichnet, daß als Chlorierungsmittel 3 bis 10 Mol eines anorganischen oder organischen Säurechlorids pro Mol der Verbindung der Formel (II) eingesetzt werden.

3. Verfahren gemäß Anspruch 1 oder 2, dadurch gekennzeichnet, das als Säurechloride Phosphor(V)-chlorid, Phosphor(III)-chlorid, Phosphorylchlorid, Thionylchlorid oder Phosgen eingesetzt werden.

4. Verfahren gemäß Anspruch 1 oder 2, dadurch gekennzeichnet, daß als Säurechloride Phosgen oder Phosphorylchlorid eingesetzt werden.

5. Verfahren gemäß einem der Ansprüche 1 bis 4, dadurch gekennzeichnet, daß die Umsetzung in der ersten Stufe bei Temperaturen von 10° C bis 180° C durchgeführt wird.

6. Verfahren gemäß Anspruch 1, dadurch gekennzeichnet, daß ein Pyridin-Derivat der Formel (II) in Mischung mit einem Verdünnungsmittel und/oder Reaktionshilfsmittel vorgelegt und Phosgen bis zum vollständigen Umsatz in oder über die Mischung geleitet wird oder Phosphorylchlorid zudosiert wird.

7. Verfahren gemäß mindestens einem der Ansprüche 1 bis 6, dadurch gekennzeichnet, daß als Säureakzeptoren Alkalimetallhydroxide, Erdalkalimetallhydroxide, Alkylihydrogencarbonate oder -alkohlate oder aliphatische, aromatische oder heterocyclische Amine eingesetzt werden.

8. Verfahren gemäß Anspruch 7, dadurch gekennzeichnet, daß als Säureakzeptoren ein oder mehrere der folgenden Säurebindemittel eingesetzt werden: Natrium- und Kaliumhydroxid, Calciumhydroxid, Natrium- und Kaliumcarbonat, Natrium-und Kaliumhydrogencarbonat, Natrium- und Kaliummethylat bzw. -ethylat, Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

9. Verfahren gemäß Anspruch 1, dadurch gekennzeichnet, daß die Umsetzungen in der zweiten Stufe bei Temperaturen von 50° C bis 100° C durchgeführt werden.

## Claims

1. Process for the preparation of chlorinated nicotinaldehydes of the general formula (I)

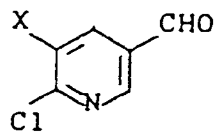

(I)

in which
X represents hydrogen or chlorine,
characterized in that, in a first step, 6-alkoxynicotinaldehydes of the general formula (II)

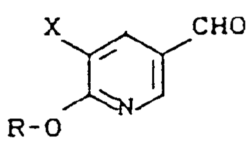

(II)

in which
X has the abovementioned meaning and
R represents alkyl,
are reacted with chlorinating agents, if appropriate in the presence of diluents and if appropriate in the presence of reaction auxiliaries, at temperatures from 0°C to 200°C and then, in a second step, the dichloromethylpyridines thus obtained of the general formula (III)

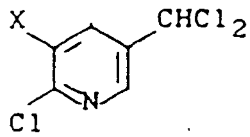

(III)

in which
X has the abovementioned meaning,
are reacted with water, if appropriate in the presence of acid acceptors, at temperatures from 0°C to 110°C.

2. Process according to Claim 1, characterized in that 3 to 10 moles of an inorganic or organic acid chloride per mole of the compound of the formula (II) are employed as chlorinating agents.

3. Process according to Claim 1 or 2, characterized in that phosphorus (V) chloride, phosphorus(III) chloride, phosphoryl chloride, thionyl chloride or phosgene are employed as acid chlorides.

4. Process according to Claim 1 or 2, characterized in that phosgene or phosphoryl chloride are employed as acid chlorides.

5. Process according to one of Claims 1 to 4, characterized in that, in the first step, the reaction is carried out at temperatures from 10°C to 180°C.

6. Process according to Claim 1, characterized in that a pyridine derivative of the formula (II) is initially introduced in a mixture with a diluent and/or reaction auxiliary and phosgene is passed into or through the mixture until conversion is complete or phosphoryl chloride is metered in.

7. Process according to at least one of Claims 1 to 6, characterized in that alkali metal hydroxides, alkaline earth metal hydroxides, alkali metal hydrogencarbonates or alkoxides or aliphatic, aromatic or heterocyclic amines are employed as acid acceptors.

8. Process according to Claim 7, characterized in that one or more of the following acid-binding agents are employed as acid acceptors:
sodium hydroxide and potassium hydroxide, calcium hydroxide, sodium carbonate and potassium carbonate, sodium hydrogencarbonate and potassium hydrogencarbonate, sodium methoxide or ethoxide and potassium methoxide or ethoxide, triethylamine, trimethylamine, dimethylaniline, dimethylbenzylamine, pyridine, 1,5-diazabicyclo-[4.3.0]-non-5-ene (DBN), 1,8-diazabicyclo-[5.4.0]-undec-7-ene (DBU) and 1,4-diazabicyclo-[2.2.2]-octane (DABCO).

9. Process according to Claim 1, characterized in that, in the second step, the reactions are carried out at temperatures from 50°C to 100°C.

## Revendications

1. Procédé de préparation d'aldéhydes nicotiniques chlorés de la formule générale (I)

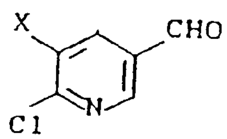

(I)

dans laquelle
X représente de l'hydrogène ou du chlore,
caractérisé en ce que l'on fait réagir, au cours d'une première étape, des aldéhydes 6-alcoxynicotiniques de formule générale (II)

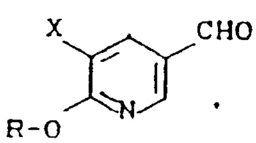

(II)

dans laquelle
X a la signification précitée et

R représente un radical alkyle,

avec des agents de chloration, éventuellement en présence de diluants et, éventuellement, en présence d'adjuvants de réaction à des températures de 0°C à 200°C et qu'on fait réagir, au cours d'une deuxième étape, les dichlorométhylpyridines de formule générale (III)

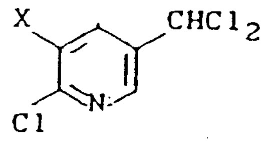

(III)

dans laquelle

X a la signification précitée,

avec de l'eau, éventuellement en présence d'accepteurs d'acides à des températures de 0°C à 110°C.

2. Procédé selon la revendication 1, caractérisé en ce que l'on utilise comme agent de chloration de 3 à 10 moles d'un chlorure d'acide inorganique ou organique pour chaque mole du composé de formule (II).

3. Procédé selon la revendication 1 ou 2, caractérisé en ce que l'on utilise comme chlorures d'acide du chlorure de phosphore(V), du chlorure de phosphore (III), du chlorure de phosphoryle, du chlorure de thionyle ou du phosgène.

4. Procédé selon la revendication 1 ou 2, caractérisé en ce que l'on utilise comme chlorures d'acide du phosgène ou du chlorure de phosphoryle.

5. Procédé selon l'une des revendications 1 à 4, caractérisé en ce que la réaction de la première étape est effectuée à des températures de 10°C à 180°C.

6. Procédé selon la revendication 1, caractérisé en ce que l'on prépare un dérivé pyridinique de formule (II) en mélange avec un diluant et/ou un adjuvant de réaction et qu'on introduit du phosgène jusqu'à réaction complète dans ou au-dessus du mélange ou qu'on y ajoute du chlorure de phosphoryle.

7. Procédé selon au moins l'une des revendications 1 à 6, caractérisé en ce que l'on utilise comme accepteurs d'acide des hydroxydes de métaux alcalins, des hydroxydes de métaux alcalino-terreux, des hydrogénocarbonates ou des alcoolates d'alkyle ou des amines aliphatiques, aromatiques ou hétérocycliques.

8. Procédé selon la revendication 7, caractérisé en ce que l'on utilise comme accepteurs caractérisé en ce que l'on utilise comme accepteurs d'acide un ou plusieurs des fixateurs d'acide suivants : hydroxydes de sodium et de potassium, hydroxyde de calcium, carbonates de sodium et de potassium, hydrogénocarbonates de sodium et de potassium, éthylates ou méthylates de sodium et de potassium, triéthylamine, triméthylamine, diméthylaniline, diméthylbenzylamine, pyridine, 1,5-diazabicyclo-[4,3,0]-nonène-5(DBN), 1,8-diazabicyclo-[5,4-0]-undécène-7(DBU) et 1,4-diazabicyclo-[2,2,2]- octane (DABCO).

9. Procédé selon la revendication 1, caractérisé en ce que l'on effectue les réactions de la deuxième étape à des températures de 50°C à 100°C.